# EUROPÄISCHE PATENTSCHRIFT

(11) **EP 2 849 717 B1**
(45) Veröffentlichungstag und Bekanntmachung des Hinweises auf die Patenterteilung: **19.04.2017**
(21) Anmeldenummer: 13718593.0
(22) Anmeldetag: 29.04.2013
(51) Int. Cl.: A61K 8/31, A61K 8/37, A61K 8/39, A61K 8/86, A61Q 19/00, A61Q 1/06

(54) **AROMATISIERTE LIPPENBUTTER**
FLAVOURED LIP BUTTER
BAUME À LÈVRES AROMATISÉ

(30) Priorität: 14.05.2012 DE 102012207989
(43) Veröffentlichungstag der Anmeldung: 25.03.2015
(73) Patentinhaber: Beiersdorf AG, 20253 Hamburg (DE)
(72) Erfinder: RESHAD, Sepideh, 22175 Hamburg (DE); DIEC, Khiet Hien, 20251 Hamburg (DE); TEICHERT, Uta, 22609 Hamburg (DE); SELLCKAU, Sabine, 22455 Hamburg (DE)
(86) Internationale Anmeldenummer: PCT/EP2013/058846
(87) Internationale Veröffentlichungsnummer: WO 2013/171056

(56) Entgegenhaltungen:
- WO-A1-94/26234
- DE-U1- 9 316 320
- DATABASE GNPD [Online] MINTEL; 31. Januar 2012 (2012-01-31), "Raspberry Lip Butter", XP002715041, Database accession no. 1718218

## Beschreibung

Die vorliegende Erfindung betrifft eine kosmetische Lippenbutter enthaltend mindestens 60 Gew.-% Vaseline, einen nichtionischen Emulgator und ein oder mehrere Aromastoffe aus der Gruppe der Ester.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren, immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Hautpflegeprodukte, in der Regel Cremes, Salben oder Lotionen, dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Neben der Reinigung und Pflege der Haut haben Kosmetika auch eine ästhetische Aufgabe. Sie sollen das äußere Erscheinungsbild des Anwenders entsprechend den jeweiligen kulturellen Vorstellungen "verbessern". Kosmetika erfüllen damit eine psychologisch-soziale Funktion, da sie die (optische) Attraktivität der Anwender erhöhen. In diesen Bereich fällt vor allen Dingen die "dekorative" Kosmetik, die mit Hilfe von auf die Haut aufgetragenen Farbstoffen das Erscheinungsbild der Anwender verändert. Indirekt haben aber auch Reinigungs- und Pflegeprodukte einen positiven Einfluss, da eine saubere, gesunde Haut dem Schönheitsideal der Menschen entspricht.

Eine besondere Form kosmetischer Zubereitungen stellen die Lippenpflegeprodukte dar. Sie dienen einerseits der Pflege der Lippenhaut und schützen bzw. regenerieren die Lippen bei Trockenheit und Rissigkeit. Andererseits dienen Lippenpflegeprodukte auch zur Dekoration, da sie den Lippen durch Glanzeffekte oder Farbe zu einem besonderen Ausdruck verhelfen.

Lippenpflegeprodukte werden sowohl in Stiftform oder als mehr oder weniger zähfließende Crememasse dargereicht. Eine besondere Form stellt die Lippenbutter dar, die eine Butter-artige Konsistenz aufweist und aus unterschiedlichen Fettkomponenten besteht.

An Lippenpflegeprodukte werden besondere Anforderungen gestellt. Sie müssen in toxikologischer Hinsicht vollkommen unbedenklich sein um jegliche Gefahr einer Vergiftung auszuschließen. Darüber hinaus müssen sie einerseits streichfähig sein, andererseits, nach dem Auftragen auf die Lippen aber dort ortsfest verbleiben ohne klebrig zu wirken. Nicht zuletzt müssen diese Produkte mikrobiologisch und thermisch über einen langen Zeitraum stabil sein.

Lippenpflegeprodukte, insbesondere cremig-softe "Lippenbutter"-Produkte, die üblicherweise in Tiegeln dargereicht werden, haben häufig den Nachteil, dass sie nur schwer aromatisiert werden können. So kommt es insbesondere bei den Geschmacksnoten Himbeere, Karamell/Pfirsich, Vanille/Macadamia bei der Herstellung der Zubereitungen zu Inhomogenitäten und Tröpfchenbildung am Boden des Mischgefäßes bzw. der Tiegel. Dieser Effekt tritt insbesondere dann auf, wenn als Aromastoffe Ester, insbesondere Acetate und Buryrate enthalten sind und wenn den Aromastoffen als Lösemittel bzw. Trägerstoff Glyceroltriacetat zugesetzt wurde.

Es war daher die Aufgabe der vorliegenden Erfindung, die Nachteile des Standes der Technik zu beseitigen und eine stabile, homogene aromatisierte Lippenbutter zu entwickeln.

Gelöst wird die Aufgabe durch eine kosmetische Lippenbutter enthaltend
a) mindestens 60 Gew.-%, bezogen auf das Gesamtgewicht der Zubereitung, Vaseline,
b) einen nichtionischen Emulgator,
c) ein oder mehrere Aromastoffe aus der Gruppe der Ester,
wobei als nichtionischer Emulgator Polyglyceryl-3 Diisostearat eingesetzt wird.

Gelöst wird die Aufgabe insbesondere durch ein Verfahren zur Herstellung einer kosmetischen Lippenbutter, dadurch gekennzeichnet, dass die Aromastoffe zusammen und gleichzeitig mit dem nichtionischen Emulgator unter Rühren zur erwärmten Vaseline bzw. Vaseline/Sheabutter Mischung zugesetzt werden, sowie durch das entsprechende Verfahrensprodukt.

Zwar kennt der Stand der Technik die DE-U-93 16 320. WO-A-94/26234 und XP002715041 (Raspberry Lip Butter) aus der gnpd-Datenbank Mintel (Record ID:1718218), doch konnten diese Offenbarungen nicht den Weg zur vorliegenden Erfindung weisen.

Es ist erfindungsgemäß vorteilhaft, wenn die kosmetische Lippenbutter Glyceroltriacetat enthält.

Dabei bezieht sich "erfindungsgemäße Lippenbutter", "erfindungsgemäß vorteilhafte Lippenbutter" etc. im Rahmen der vorliegenden Beschreibung immer sowohl auf die erfindungsgemäße Zubereitung, das erfindungsgemäße Verfahren und das nach dem erfindungsgemäßen Verfahren hergestellte Verfahrensprodukt, auch wenn dies nicht explizit erwähnt sein sollte.

Es ist erfindungsgemäß vorteilhaft, wenn die Zubereitung Glyceroltriacetat in einer Menge von 0,1 bis 1 Ges.-%. bezogen auf das Gesamtgewicht der Zubereitung, beträgt.

Desweiteren ist es erfindungsgemäß vorteilhaft, die Zubereitung den nichtionischen Emulgator in einer Menge von 0,001 bis 5 Gew.-%, bezogen auf das Gesamtgewicht der Zubereitung, enthält.

Erfindungsgemäß vorteilhafte Ausführungsformen der vorliegenden Erfindung sind dadurch gekennzeichnet, dass die Zubereitung Sheabutter enthält.

Dabei ist es erfindungsgemäß bevorzugt, wenn die Menge an Vaseline und Sheabutter in der Zubereitung mindestens 75 Gew.-%, bezogen auf das Gesamtgewicht der Zubereitung, beträgt.

Neben Sheabutter und Vaseline, kann die erfindungsgemäße Zubereitung weitere lipophile Bestandteile enthalten, insbesondere Wachse (beispielsweise Candelillawachs, Carnaubawachs, Bienenwachs, Schellackwachs, Beerenwachs, Hydriertes Jojobawachs, Wollwachs, Zuckerrohrwachs, Reiskeimwachs), nicht zu den Aromastoffen gehörende Esteröle, Mineralöle, Triglyceride (beispielsweise Capryl/Caprinsäure Triglycerid) sowie Pflanzenöle, beispielsweise Jojobaöl und Mandelöl.

Es ist erfindungsgemäß von Vorteil, wenn die Aromastoffe aus der Gruppe der Ester gewählt werden aus der Gruppe der Acetate und Butyrate.
Dabei ist es erfindungsgemäß bevorzugt, wenn als Aromastoff Isopentylacetat eingesetzt wird.

Erfindungsgemäß vorteilhafte Ausführungsformen der vorliegenden Erfindung sind auch dadurch gekennzeichnet, dass die Zubereitung neben den Aromastoffen aus der Gruppe der Ester weitere Aromastoffe enthält.

Dabei ist es erfindungsgemäß vorteilhaft, wenn die Gesamtmenge an Aromastoffen in der Zubereitung von 0,001 bis 3 Gew.-%, bezogen auf das Gesamtgewicht der Zubereitung, beträgt.

Erfindungsgemäß vorteilhafte Ausführungsformen sind auch dadurch gekennzeichnet, dass die die Zubereitung Antioxidantien enthält.

Als erfindungsgemäß bevorzugte Antioxidantien werden dabei BHT (Butylhydroxytoluol), Tocopherol und Tocopherolacetat angesehen.

Erfindungsgemäß vorteilhaft ist es, wenn die Zubereitung frei ist von Polyethylenglycolen und deren Derivaten.

Erfindungsgemäß vorteilhafte Ausführungsformen zeichnen sich darüber hinaus dadurch aus, dass die Zubereitung frei ist von Parabenen.

Nicht zuletzt sind die erfindungsgemäßen Zubereitungen erfindungsgemäß vorteilhaft frei ist von Silikonölen oder-wachsen.

Die erfindungsgemäßen Zubereitungen können darüber hinaus erfindungsgemäß vorteilhaft Farb- und Parfümstoffe enthalten, wobei hierfür alle für die Lippenkosmetik bekannten Farb- und Parfümstoffe eingesetzt werden können.

Erfindungsgemäße Farbpigmente können organischen und anorganischen Ursprungs sein, wie beispielsweise organische vom Azo-Typ, Indigoide, Triphenylmethan-artige, Anthrachinone, und Xanthin Farbstoffe, die als D&C and FD&C blues, browns, greens, oranges, reds, yellows bekannt sind. Anorganische Pigmente bestehen aus unlöslichen Salzen von zertifizierten Farbstoffen, die als Lakes oder Eisenoxide bezeichnet werden.

Beispielsweise können Barium lakes, calcium lakes, aluminum lakes, titandioxide, mica and iron oxides Verwendung finden. Als Al-Salze sind z.B Red 3 Aluminum Lake, Red 21 Aluminum Lake, Red 27 Aluminum Lake, Red 28 Aluminum Lake, Red 33 Aluminum Lake, Yellow 5 Aluminum Lake, Yellow 6 Aluminum Lake, Yellow 10 Aluminum Lake, Orange 5 Aluminum Lake, Blue 1 Aluminum Lake und Kombinationen einsetzbar.

Als Eisenoxide oder -oxidhydrate sind z.B. cosmetic yellow oxide C22-8073 (Sunchemical) cosmetic yellow oxide C33-1700 (Sunchemical), cosmetic brown oxide C33-115 (Sunchemical), cosmetic iron oxide red C33-2199 (Sunchemical), cosmetic russet oxide C33-8075 (Sunchemical), cosmetic iron oxide black C33-5000 (Sunchemical), als Titandioxide z.B. Kronos 1171 (Kronos), C47-051 Cosmetic White (Sunchemical) bekannt und gegeben falls vorteilhaft.

Ebenso ist es bevorzugt, wenn diese Zubereitungen einen Gehalt an anorganischen Pigmenten gewählt aus der Gruppe der Metalloxide und/oder anderen in Wasser schwerlöslichen oder unlösliche Metallverbindungen, bevorzugt Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄) aufweisen.

Erfindungsgemäß vorteilhaft enthält die erfindungsgemäße Zubereitung 0-5 Gewichts-% Perlglanzpigmente.

Dazu zählen natürliche Perlglanzpigmente, wie z. B.
▪ "Fischsilber" (Guanin/Hypoxanthin-Mischkristalle aus Fischschuppen) und
▪ "Perlmutt" (vermahlene Muschelschalen),
monokristalline Perlglanzpigmente wie z. B. Bismuthoxychlorid (BiOCI),
Schicht-Substrat Pigmente: z. B. Glimmer / Metalloxid
Vorteilhaft sind ferner beispielsweise die folgenden Perlglanzpigmentarten auf Basis von Glimmer/Metalloxid:

| ***Gruppe*** | Belegung / Schichtdicke | Farbe |
|---|---|---|
| **Silberweiße Perlglanzpigmente** | TiO₂: 40 - 60 nm | silber |
| **Interferenzpigmente** | TiO₂: 60 - 80 nm | gelb |
| | TiO₂: 80 - 100 | rot |
| | TiO₂: 100 - 140 nm | blau |
| | TiO₂: 120 - 160 nm | grün |
| **Farbglanzpigmente** | Fe₂O₃ | bronze |
| | Fe₂O₃ | kupfer |
| | Fe₂O₃ | rot |
| | Fe₂O₃ | rotviolett |
| | Fe₂O₃ | rotgrün |
| | Fe₂O₃ | schwarz |
| **Kombinationspigmente** | TiO₂ / Fe₂O₃ | Goldtöne |
| | TiO₂ / Cr₂O₃ | grün |
| | TiO₂ / Berliner Blau | tiefblau |
| | TiO₂ / Carmin | rot |

Als besonders bevorzugt sind die Pigmente der Firmen Costenoble (Cloisonne-Typ, Flamenco-Typ, Low Lustre-Typ), Merck (Colorona-Typen, Microna-Typ, Timiron-Typ, Colorona, Ronasphere), Les Colornats Wacker (Covapure, Vert oxyde de Chrome), Cadre (Colorona, Sicopearl), BASF (Sicopearl, Sicovit), Rona (Colorona) bekannt.

Es kann darüber hinaus von Vorteil sein, gänzlich auf ein Substrat wie Glimmer zu verzichten. Besonders bevorzugt sind Perlglanzpigmente, welche unter der Verwendung von SiO₂ hergestellt werden, z.B. solche wie von Merck unter dem Namen Xirona erhältlichen. Solche Pigmente, die auch zusätzlich gonichromatische Effekte haben können, sind z. B. unter dem Handelsnamen Sicopearl Fantastico bei der Firma BASF erhältlich.

Vorteilhaft können Pigmente auf Basis von synthetischem Glimmer (Synthetic Fluorophlogopite) der Firma Sunchemical. Diese sind unter dem Namen SunShine erhältlich.

Weiterhin vorteilhaft können Pigmente der Firma Engelhard / Mearl auf Basis von Calcium Natrium Borosilikat, die mit Titandioxid beschichtet sind, eingesetzt werden. Diese sind unter dem Namen Reflecks erhältlich. Sie weisen durch ihrer Partikelgröße von 40 - 180 µm zusätzlich zu der Farbe einen Glitzereffekt auf.

Weiterhin vorteilhaft können Pigmente der Firma Merck auf Basis von Calcium Aluminium Borosilikat, die mit Titandioxid beschichtet sind, eingesetzt werden. Diese sind unter dem Namen Ronastar erhältlich.

Die erfindungsgemäße kosmetische Zubereitung ist erfindungsgemäß vorteilhaft dadurch gekennzeichnet, dass die Zubereitung eine Konsistenz, gemessen mit Fliggemeter, von größer 30 bei 20°C und 1,013 bar aufweist. Das Fliggemeter ist ein Konsistenzmessgerät, das in der deutschen Offenlegungsschrift DE 2909087 beschrieben ist.

Die erfindungsgemäße Zubereitung kann erfindungsgemäß weitere Bestandteile enthalten. So ist es erfindungsgemäß vorteilhaft, wenn die erfindungsgemäße Zubereitung als weitere Bestandteile eine oder mehrere Verbindungen gewählt aus der Gruppe der Konservierungsmittel, UV-Lichtschutzfilter, Filmbildner, Vitamin-A-Palmitat, Vitamin-E-Acetat, Allantoin, Panthenol, α-Bisabolol, Lecithin, Ceramide , Collagene, Ubiquinone, Ginseng-Extrakt, Traubenkernöl, Hopfenwasser, Klettenfrucht-Extrakt, Sonnenblumenextrakt, sowie pflegende Öle auf pflanzlicher Basis enthält.

Die nachfolgenden Beispiele sollen die vorliegende Erfindung verdeutlichen. Die Zahlenwerte in den Beispielen bedeuten Gewichtsprozente, bezogen auf das Gesamtgewicht der Zubereitungen.

### Beispiele

| | 1 | 2 | 3 | 4 | 5 | 6 | 7 | 8 | 9 | 10 |
|---|---|---|---|---|---|---|---|---|---|---|
| Cera Microcristallina + Paraffinum Liquidum | 93,59 | 93,54 | 92,59 | 93,19 | 92,13 | 94 | 91,65 | 93,89 | 85 | 85 |
| Butyrospermum Parkii Butter | 0,9 | 1,2 | 1 | 0,5 | 0,75 | 0,3 | 2 | 0,6 | 6 | 2 |
| Ricinus Communis Seed Oil | 0,5 | 0,5 | | 1,4 | 1 | 1,3 | 1,5 | 0,5 | 4,8 | 2 |
| Prunus Amygdalus Dulcis Oil | 0,5 | 0,7 | | | 0,5 | 0,5 | | | | |
| Simmondsia Chinensis Seed Oil | | | 0,5 | | | | | | 0,1 | 2 |
| Persea Gratissima Oil | | | | 0,4 | 0,5 | | 0,5 | | | |
| Vitis Vinifera Seed Oil | | | | 0,4 | | | | 0,75 | | |
| Cera Alba | | | | | | | 0,25 | 0,25 | | |
| Polyglyceryl-3 Diisostearate | 2,5 | 2,5 | 3 | 2,7 | 2 | 2 | 2 | 2,5 | 2,5 | 3 |
| PEG-45/Dodecyl Glycol Copolymer | | | | | 0,5 | 0,5 | | | | |
| Caprylic/Capric Triglyceride | | | | | | | 0,5 | 0,2 | | |
| Octyldodecanol | 0,5 | | 0,5 | | 1,5 | | | | | 1 |
| Polydecene | | | | | | | | | 0,2 | 3,5 |
| Panthenol | | | | 0,1 | 0,1 | | | | | |
| Glycerin | 0,2 | 0,2 | 0,75 | | | 0,1 | 0,1 | 0,4 | | |
| Glyceryl Glucoside | | 0,2 | | | | 0,1 | | | | |
| Tocopheryl Acetate | | | | 0,1 | | | | | 0,2 | |
| Cera Carnauba | | | 0,2 | | | | | | | |
| Flavor | 1 | 1 | 1,2 | 1 | 1 | 1 | 1 | 0,8 | 1 | 1 |
| Cl 77891 | 0,3 | 0,155 | 0,25 | 0,2 | | 0,15 | 0,5 | 0,1 | 0,2 | 0,2 |
| Cl 77492 | 0,01 | 0,005 | | | 0,02 | | | | | |
| Cl 45380 | | | | | | 0,05 | | | | |
| Cl 15985 | | | | 0,005 | | | | 0,01 | | 0,1 |
| Cl 15850 | | | 0,01 | 0,005 | | | | | | 0,2 |
| | 100 | 100 | 100 | 100 | 100 | 100 | 100 | 100 | 100 | 100 |

## Patentansprüche

1. Kosmetische Lippenbutter enthaltend
a) mindestens 60 Gew.-%, bezogen auf das Gesamtgewicht der Zubereitung, Vaseline,
b) einen nichtionischen Emulgator,
c) ein oder mehrere Aromastoffe aus der Gruppe der Ester,
wobei als nichtionischer Emulgator Polyglyceryl-3 Diisostearat eingesetzt wird.

2. Kosmetische Lippenbutter nach Anspruch 1, **dadurch gekennzeichnet, dass** die Zubereitung Glyceroltriacetat enthält.

3. Kosmetische Lippenbutter nach einem der vorhergehenden Ansprüche, **dadurch gekennzeichnet, dass** die Zubereitung Glyceroltriacetat in einer Menge von 0,1 bis 1 Gew.-%, bezogen auf das Gesamtgewicht der Zubereitung, beträgt.

4. Kosmetische Lippenbutter nach einem der vorhergehenden Ansprüche, **dadurch gekennzeichnet, dass** die Zubereitung den nichtionischen Emulgator in einer Menge von 0,001 bis 5 Gew.-%, bezogen auf das Gesamtgewicht der Zubereitung, enthält.

5. Kosmetische Lippenbutter nach einem der vorhergehenden Ansprüche, **dadurch gekennzeichnet, dass** die Zubereitung Sheabutter enthält.

6. Kosmetische Lippenbutter nach einem der vorhergehenden Ansprüche, **dadurch gekennzeichnet, dass** die Menge an Vaseline und Sheabutter in der Zubereitung mindestens 75 Gew.-%, bezogen auf das Gesamtgewicht der Zubereitung, beträgt.

7. Kosmetische Lippenbutter nach einem der vorhergehenden Ansprüche, **dadurch gekennzeichnet, dass** die Aromastoffe aus der Gruppe der Ester gewählt werden aus der Gruppe der Acetate und Butyrate.

8. Kosmetische Lippenbutter nach einem der vorhergehenden Ansprüche, **dadurch gekennzeichnet, dass** als Aromastoff Isopentylacetat eingesetzt wird.

9. Kosmetische Lippenbutter nach einem der vorhergehenden Ansprüche, **dadurch gekennzeichnet, dass** die Zubereitung neben den Aromastoffen aus der Gruppe der Ester weitere Aromastoffe enthält.

10. Kosmetische Lippenbutter nach einem der vorhergehenden Ansprüche, **dadurch gekennzeichnet, dass** die Gesamtmenge an Aromastoffen in der Zubereitung von 0,01 bis 3 Gew.-%, bezogen auf das Gesamtgewicht der Zubereitung, beträgt.

11. Verfahren zur Herstellung einer kosmetischen Lippenbutter nach einem der vorhergehenden Ansprüche, **dadurch gekennzeichnet, dass** die Aromastoffe zusammen und gleichzeitig mit dem nichtionischen Emulgator unter Rühren zur erwärmten Vaseline bzw. Vaseline/Sheabutter Mischung zugesetzt werden.

12. Lippenbutter hergestellt nach einem Verfahren nach Anspruch 11.

## Claims

1. Cosmetic lip butter comprising
a) at least 60% by weight Vaseline, based on the total weight of the preparation,
b) a non-ionic emulsifier,
c) one or more aroma substances from the group of esters,
wherein polyglyceryl-3 diisostearate is used as non-ionic emulsifier.

2. Cosmetic lip butter according to Claim 1, **characterized in that** the preparation comprises glycerol triacetate.

3. Cosmetic lip butter according to either of the preceding claims, **characterized in that** the preparation accounts for glycerol triacetate in an amount from 0.1 to 1% by weight, based on the total weight of the preparation.

4. Cosmetic lip butter according to any of the preceding claims, **characterized in that** the preparation comprises the non-ionic emulsifier in an amount from 0.001 to 5% by weight, based on the total weight of the preparation.

5. Cosmetic lip butter according to any of the preceding claims, **characterized in that** the preparation comprises shea butter.

6. Cosmetic lip butter according to any of the preceding claims, **characterized in that** the amount of Vaseline and shea butter in the preparation is at least 75% by weight, based on the total weight of the preparation.

7. Cosmetic lip butter according to any of the preceding claims, **characterized in that** the aroma substances are selected from the group of esters of the group of acetates and butyrates.

8. Cosmetic lip butter according to any of the preceding claims, **characterized in that** the aroma substance used is isopentyl acetate.

9. Cosmetic lip butter according to any of the preceding claims, **characterized in that**, in addition to the aroma substances from the group of esters, the preparation comprises further aroma substances.

10. Cosmetic lip butter according to any of the preceding claims, **characterized in that** the total amount of aroma substances in the preparation is from 0.01 to 3% by weight, based on the total weight of the preparation.

11. Method for preparing a cosmetic lip butter according to any of the preceding claims, **characterized in that** the aroma substances together and simultaneously with the non-ionic emulsifier are added with stirring to the heated Vaseline or Vaseline/shea butter mixture.

12. Lip butter prepared by a method according to Claim 11.

## Revendications

1. Baume à lèvres cosmétique, contenant :
a) au moins 60 % en poids, par rapport au poids total de la préparation, de vaseline,
b) un émulsifiant non ionique,
c) une ou plusieurs substances aromatisantes du groupe des esters,
du 3-diisostéarate de polyglycéryle étant utilisé en tant qu'émulsifiant non ionique.

2. Baume à lèvres cosmétique selon la revendication 1, **caractérisé en ce que** la préparation contient du triacétate de glycérol.

3. Baume à lèvres cosmétique selon l'une quelconque des revendications précédentes, **caractérisé en ce que** la préparation contient du triacétate de glycérol en une quantité de 0,1 à 1 % en poids, par rapport au poids total de la préparation.

4. Baume à lèvres cosmétique selon l'une quelconque des revendications précédentes, **caractérisé en ce que** la préparation contient l'émulsifiant non ionique en une quantité de 0,001 à 5 % en poids, par rapport au poids total de la préparation.

5. Baume à lèvres cosmétique selon l'une quelconque des revendications précédentes, **caractérisé en ce que** la préparation contient du beurre de karité.

6. Baume à lèvres cosmétique selon l'une quelconque des revendications précédentes, **caractérisé en ce que** la quantité de vaseline et de beurre de karité dans la préparation est d'au moins 75 % en poids, par rapport au poids total de la préparation.

7. Baume à lèvres cosmétique selon l'une quelconque des revendications précédentes, **caractérisé en ce que** les substances aromatisantes du groupe des esters sont choisies dans le groupe des acétates et des butyrates.

8. Baume à lèvres cosmétique selon l'une quelconque des revendications précédentes, **caractérisé en ce que** de l'acétate d'isopentyle est utilisé en tant que substance aromatisante.

9. Baume à lèvres cosmétique selon l'une quelconque des revendications précédentes, **caractérisé en ce que** la préparation contient d'autres substances aromatisantes en plus des substances aromatisantes du groupe des esters.

10. Baume à lèvres cosmétique selon l'une quelconque des revendications précédentes, **caractérisé en ce que** la quantité totale de substances aromatisantes dans la préparation est de 0,01 à 3 % en poids, par rapport au poids total de la préparation.

11. Procédé de fabrication d'un baume à lèvres cosmétique selon l'une quelconque des revendications précédentes, **caractérisé en ce que** les substances aromatisantes sont ajoutées conjointement et simultanément avec l'émulsifiant non ionique sous agitation à la vaseline ou au mélange vaseline/beurre de karité chauffé.

12. Baume à lèvres fabriqué par un procédé selon la revendication 11.
